# EUROPEAN PATENT APPLICATION

(11) **EP 3 272 865 A1**
(43) Date of publication of application: **24.01.2018**
(21) Application number: 16768715.1
(22) Date of filing: 18.03.2016
(51) Int. Cl.: C12N 15/00, C12Q 1/68, C12N 9/12, C12N 9/22

(54) **HIGH-SENSITIVITY METHOD FOR DETECTING TARGET NUCLEIC ACID**

(30) Priority: 20.03.2015 JP 2015058270
(71) Applicant: Takara Bio Inc., Kusatsu-shi Shiga 525-0058 (JP)
(72) Inventor: UEMORI, Takashi, Kusatsu-shi Shiga 525-0058 (JP); SAGARA, Takehiro, Kusatsu-shi Shiga 525-0058 (JP); INOUE, Koichi, Kusatsu-shi Shiga 525-0058 (JP)
(74) Representative: Schnappauf, Georg
(86) International application number: PCT/JP2016/058852
(87) International publication number: WO 2016/152812

(57) **Abstract**

Provided are: a method with which it is possible to suppress nucleic-acid amplification of non-target nucleic acids and to selectively nucleic-acid amplify a target nucleic acid in a sample in which a large amount of non-target nucleic acids and a rare target nucleic acid are present in mixture; a method for detecting at high sensitivity rare mutant genes, the method combining this nucleic-acid amplification method and a specific real-time method for detecting the target nucleic acid; and a composition and kit for this method.

## Description

### Technical Field

The present invention relates to a high-sensitivity detection method of a target nucleic acid comprising a combination of at least one oligonucleotide which selectively suppresses nucleic acid amplification of a non-target nucleic acid and a polypeptide having a mismatch endonuclease activity.

### Background Art

It is known that there are hereditary or acquired diseases related to genetic mutations including base substitution, deletion and insertion. Among these mutations, there are some mutations are directly related to the diseases and some mutations are correlated to risk and/or prognosis of the diseases. Among these diseases, for diseases in which a wild-type gene changes to a mutant-type gene or cells having a mutant-type gene are increased with the progress of diseases, detection of the existence of the mutant-type gene is important. Examples of the acquired diseases accompanied by a genetic mutation and examples of the genetic mutation related to the diseases include lung cancer and EGFR gene mutation or K-ras gene mutation; lung squamous cell carcinoma and DDR2 gene mutation; colorectal cancer and K-ras gene mutation, PIK3CA gene mutation or B-raf gene mutation; and gastric cancer and Kit gene mutation or PDGFR gene mutation.

There are also opposite cases to the above-mentioned cases. For example, during therapy process for an acquired disease accompanied by a genetic mutation, a decrease of the mutant-type gene and an increase of the wild-type gene are monitored. As an example, detection of minimal residual disease is mentioned.

As a method of analyzing gene mutations, a restriction fragment length polymorphism method (RFLP method) has been conventionally used. However, the RFLP method needs cumbersome manipulation and a long time. In addition, the RFLP method may not be often applied depending on a nucleotide sequence near a single nucleotide polymorphism (SNP) site. Thus it has been pointed out that the RFLP method has many problems for clinical laboratory use. In recent years, a variety of simpler and versatile means including an Invader method, a TaqMan PCR method, a single base extension method, a pyrosequencing method, and an exonuclease cycling assay method have been developed. However, in the case of using conventional PCR, when a small amount of a mutant-type gene commingling with the wild-type gene is less than 5% of the amount of the wild-type gene, amplification reaction reaches a plateau before a target nucleic acid from the mutant-type gene reaches a detectable amount, and therefore the target nucleic acid cannot be fully detected.

Thus, the PCR method is a very useful tool for detecting a mutant-type gene as a target nucleic acid. However, in the PCR method, annealing between amplified products increasingly competes with annealing of primers as the concentration of the amplified product increases, and finally the primers cannot anneal and the reaction reaches a plateau. As a result, amplified fragments from the target nucleic acid containing SNP or short deletion or insertion anneal with amplified fragments from a non-target nucleic acid which is present in a large excess amount. Thus when the amplification reaction reaches the plateau, an abundance ratio of the amplified fragments from the target nucleic acid falls below the abundance ratio before the initiation of the reaction. Therefore, in order to detect a small amount of SNP or deletion or insertion in the same region, it is necessary to specifically amplify a nucleic acid from a mutant-type gene while suppressing amplification of a nucleic acid from the wild-type gene.

As examples of a method for selectively amplifying a nucleic acid from a mutant-type gene while suppressing amplification of a nucleic acid from the wild-type gene, an enriched PCR method (non-patent literature 1) and a restriction endonuclease-mediated selective PCR method (non-patent literature 2 and 3 and patent literature 1) have been reported. In the methods, a recognition/cleavage site for a restriction enzyme is introduced within a primer containing a nucleotide sequence of a mutated site in a gene of interest, and thereby the mutant-type gene is selectively amplified while amplification of the wild-type gene is suppressed. However, these methods require selecting a restriction enzyme capable of recognizing and cleaving a mutated site in a gene of interest to be analyzed, and in addition, a gene to be recognized and cleaved by the restriction enzyme must be the wild-type gene. In addition, the restriction enzyme must not be inactivated during cycles of PCR. There is a problem that only limited restriction enzymes meet these requirements, and subject wild-type genes and mutant-type genes to which the methods are applicable are limited.

As another method for amplifying a nucleic acid from a wild-type gene and a nucleic acid from a mutant-type gene, a mutant enrichment with 3'-modified oligonucleotides (MEMO) method (non-patent literature 4 and patent literature 2) has been suggested. The method uses a 3'-terminal blocking primer containing a mutated site in a gene of interest. The 3'-terminal blocking primer is characterized by 100% match with a wild-type gene and mismatch with a mutant-type gene. By use of the 3'-terminal blocking primer, in nucleic acid amplification of the wild-type gene, annealing and extension of primers for amplification are inhibited. On the other hand, in nucleic acid amplification of the mutant-type gene, annealing and extension of primers for amplification are not inhibited due to instability caused by the mismatch between the mutant-type gene and the 3'-terminal blocking primer. As a result, the nucleic acid amplification of the mutant-type gene is prioritized. Detection in the method is performed by melting curve analysis or sequencing. However, this method requires hybridizing the 3'-terminal blocking primer so as to inhibit only the primer-extension reaction of the wild-type gene, and requires strictly controlling temperature during the nucleic acid amplification reaction. In addition, the initial amount of the wild-type gene present in a large amount in the initial sample is maintained, and unexpected amplification of a nucleic acid from the wild-type gene cannot be decreased.

In more recent years, mutation detection methods comprising use of a heat-resistant mismatch endonuclease (patent literature 3) have been reported. However, these methods still have problems in order to suppress nucleic acid amplification of a wild-type gene and selectively amplify a mutant-type gene to properly detect only the presence of the mutant-type gene.

### Citation List

### Patent Literature

Patent Literature 1: WO 1996/32500
Patent Literature 2: US 2013/0149695 A
Patent Literature 3: WO 2014/142261

### Non Patent Literature

Non-Patent Literature 1: Leukemia, Vol.5, No.2, pp. 160-161, 1991
Non-Patent Literature 2: Oncogene, Vol.6, No.6, pp. 1079-1083, 1991
Non-Patent Literature 3: American Journal of Pathology, Vol.153, pp. 373-379, 1998
Non-Patent Literature 4: The Journal of Molecular Diagnostics, Vol.13, pp. 657-668, 2011

### Summary of Invention

### Technical Problems

An objective of the present invention is to provide a high-sensitivity detection method of a target nucleic acid which is present in a very small amount relative to a non-target nucleic acid.

### Solution to Problems

The present inventors analyzed the property of a polypeptide having a mismatch endonuclease activity that recognizes and cleaves a mismatch site, in particular a correlation between the kind or position of a mismatch and a cleaving activity. As a result, the present inventors found a method of cleaving any one of two nucleic acids which differ by at least one base in their sequences without being limited by the kind of the different base. In addition, the present inventors found a method of selectively amplifying a rare target nucleic acid, which comprises treating a sample containing a mixture of a large amount of non-target nucleic acid and the rare target nucleic acid by the above-mentioned cleavage method, thereby selectively cleaving the non-target nucleic acid, and thus enabling to selectively amplify the target nucleic acid. In addition, the present inventors found a high-sensitivity detection method of a target nucleic acid comprising the above-mentioned nucleic acid amplification method. Thus the present invention was completed.

Specifically, the first aspect of the present invention relates to a method of selectively cleaving a non-target nucleic acid in a sample containing a target nucleic acid and the non-target nucleic acid having a region of a nucleotide sequence homologous to the target nucleic acid, the method comprising a step of bringing the sample into contact with:
(i) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid; and
(ii) a polypeptide having a mismatch endonuclease activity.

In the first aspect of the present invention, the non-target nucleic acid may have a nucleotide sequence which differs from the nucleotide sequence of the target nucleic acid by base substitution, and the oligonucleotide may form at least one mismatch when the oligonucleotide is hybridized with the non-target nucleic acid and may form a mismatch corresponding to the at least one mismatch and at least another mismatch when the oligonucleotide is hybridized with the target nucleic acid. Further, the oligonucleotide may form one mismatch when the oligonucleotide is hybridized with the non-target nucleic acid and may form a mismatch corresponding to the one mismatch and another mismatch when the oligonucleotide is hybridized with the target nucleic acid. Further, the two mismatches formed when the oligonucleotide is hybridized with the target nucleic acid are preferably located contiguously or at an interval of not more than 5 bases.

In another embodiment of the first aspect of the present invention, the non-target nucleic acid has a nucleotide sequence which differs from the nucleotide sequence of the target nucleic acid by base insertion, and the oligonucleotide forms at least one mismatch when the oligonucleotide is hybridized with a region containing the insertion in the non-target nucleic acid.

In a further embodiment, the non-target nucleic acid has a nucleotide sequence which differs from the nucleotide sequence of the target nucleic acid by base deletion, and the oligonucleotide forms at least one mismatch when the oligonucleotide is hybridized with a region containing the deletion in the non-target nucleic acid.

In the first aspect of the present invention, the polypeptide having a mismatch endonuclease activity may be a polypeptide derived from a heat-resistant microorganism or a mutant thereof. In addition, the present invention can be performed in the presence of an acidic high molecular substance. Further, the present invention may be performed in combination with use of a proliferating cell nuclear antigen (PCNA).

The second aspect of the present invention relates to a method of selectively amplifying a target nucleic acid in a sample containing the target nucleic acid and a non-target nucleic acid having a region of a nucleotide sequence homologous to the target nucleic acid, the method comprising:
(1) a step of cleaving the non-target nucleic acid in the sample by the method of the first aspect of the present invention; and
(2) a step of amplifying the target nucleic acid.

In the second aspect of the present invention, the amplification of the target nucleic acid can be performed by PCR.

The third aspect of the present invention relates to a method of selectively detecting a target nucleic acid in a sample containing the target nucleic acid and a non-target nucleic acid having a region of a nucleotide sequence homologous to the target nucleic acid, the method comprising:
(1) a step of selectively amplifying the target nucleic acid by the method of the second aspect of the present invention; and
(2) a step of detecting the target nucleic acid simultaneously with or after step (1).

In the third aspect of the present invention, the detection of the target nucleic acid can be performed by a cycling probe method or a TaqMan (registered trademark) probe method.

The fourth aspect of the present invention relates to a composition for the second aspect and third aspect of the present invention, containing:
(a) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid; and
(b) at least one pair of oligonucleotide primers;
(c) a polypeptide having a mismatch endonuclease activity; and
(d) a polypeptide having a DNA polymerase activity.

The fifth aspect of the present invention relates to a kit for the second aspect and third aspect of the present invention, containing:
(a) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid; and
(b) at least one pair of oligonucleotide primers;
(c) a polypeptide having a mismatch endonuclease activity; and
(d) a polypeptide having a DNA polymerase activity.

In the fourth and fifths aspects of the present invention, the composition and the kit may further contain an acidic high molecular substance. In the present invention, the polypeptide having a mismatch endonuclease activity and the polypeptide having a DNA polymerase activity may be a heat-resistant polypeptide or a mutant thereof. The composition and the kit may further contain a proliferating cell nuclear antigen (PCNA).

The sixth aspect of the present invention relates to a method of selectively cleaving, in the presence of an acidic high molecular substance, a non-target nucleic acid in a sample containing a target nucleic acid and the non-target nucleic acid having a nucleotide sequence which differs from the nucleotide sequence of the target nucleic acid by at least one base, the method comprising a step of bringing the sample into contact with:
(i) the acidic high molecular substance;
(ii) a polypeptide having a mismatch endonuclease activity; and
(iii) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid.

In the sixth aspect of the present invention, the polypeptide having a mismatch endonuclease activity may be a heat-resistant polypeptide or a mutant thereof. Further, the present invention may be performed in combination with use of a proliferating cell nuclear antigen (PCNA).

### Effects of the Invention

According to the present invention, for a sample containing a mixture of a large amount of a non-target nucleic acid and a rare target nucleic acid, a method of suppressing nucleic acid amplification of the non-target nucleic acid and selectively amplifying the target nucleic acid is provided. In addition, a method comprising a combination of the above-mentioned nucleic acid amplification method and a specific real-time detection method of a target nucleic acid, thereby enabling to detect a rare mutant-type gene with high sensitivity is provided.

### Brief Description of Drawings

Fig. 1 shows real-time detection results of samples containing a non-target nucleic acid and a target nucleic acid at each abundance ratio prepared in Example 1.
Fig. 2 shows real-time detection results of samples containing a non-target nucleic acid and a target nucleic acid at each abundance ratio prepared in Example 2.
Fig. 3.A shows real-time detection results showing an effect of an acidic high molecular substance (in the absence of sodium alginate) on the cleavage method of the present invention in Example 3.

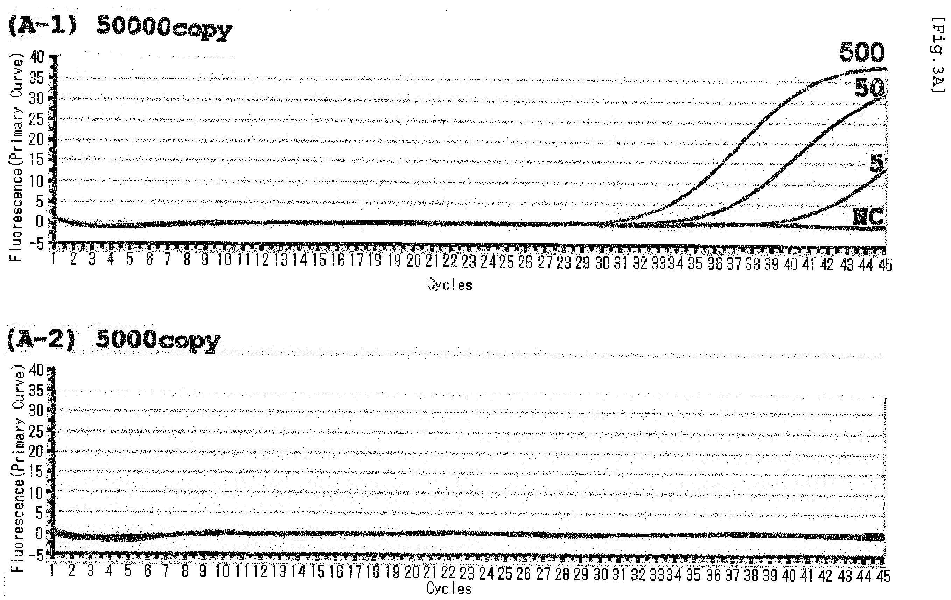

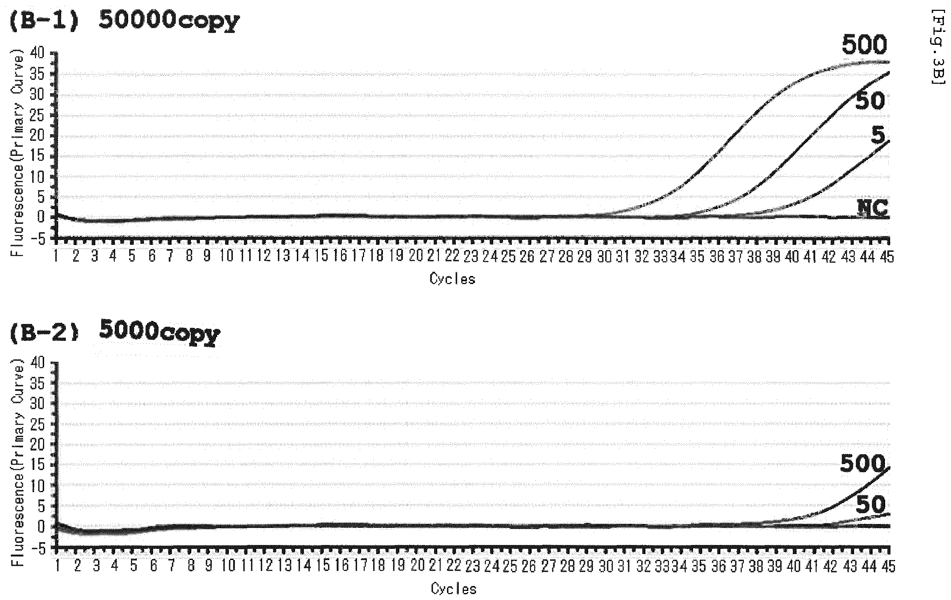

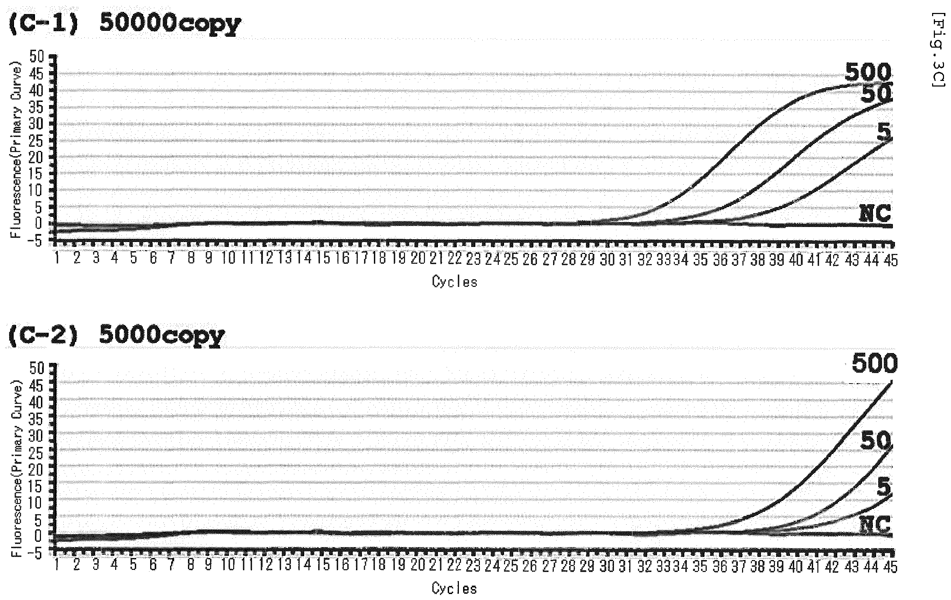

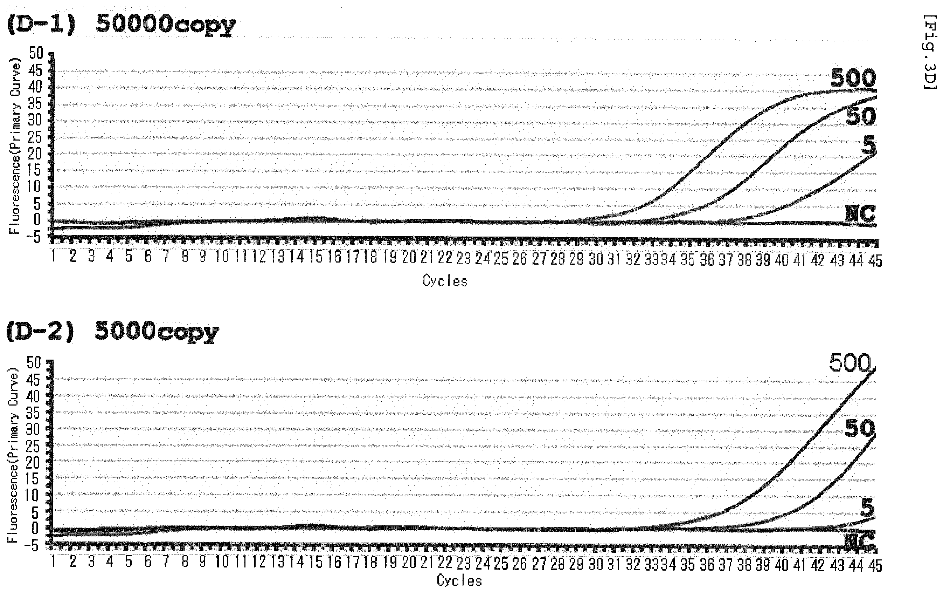

Fig. 3.B shows real-time detection results showing an effect of an acidic high molecular substance (in the presence of 56 µg/ml sodium alginate) on the cleavage method of the present invention in Example 3.
Fig. 3.C shows real-time detection results showing an effect of an acidic high molecular substance (in the presence of 140 µg/ml sodium alginate) on the cleavage method of the present invention in Example 3.
Fig. 3.D shows real-time detection results showing an effect of an acidic high molecular substance (in the presence of 168 µg/ml sodium alginate) on the cleavage method of the present invention in Example 3.

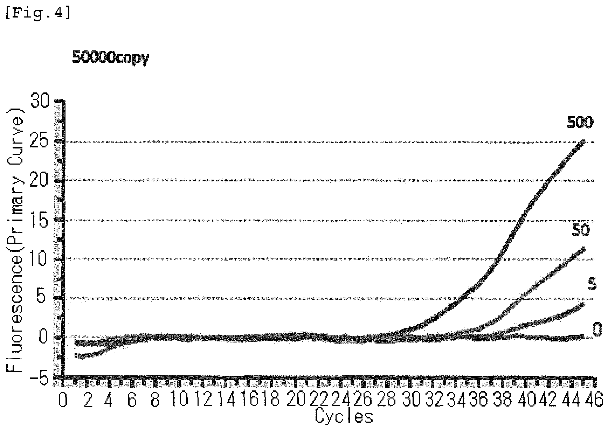

Fig. 4 shows real-time detection results of samples containing a non-target nucleic acid and a target nucleic acid at each abundance ratio prepared in Example 5.

### Mode for Carrying Out the Invention

In the present invention, the mismatch refers to base pairings different from Watson-Crick base pairs present in double-stranded nucleic acids, in other words, binding of bases in combinations other than base pairings of G (guanine base) - C (cytosine base), and A (adenine base) - T (thymine base) or U (uracil base).

In the present invention, the target nucleic acid refers to any nucleic acid which is desired to be detected (for example, a nucleic acid constituting a naturally occurring gene, and an artificially prepared nucleic acid). The present invention is useful for distinguishing the target nucleic acid from a non-target nucleic acid having a nucleotide sequence similar to the nucleotide sequence of the target nucleic acid. The target nucleic acid and the non-target nucleic acid in the present invention are preferably, but not limited to, DNA.

Examples of the target nucleic acid and the non-target nucleic acid include, but not limited to, a combination of a nucleic acid from a mutant-type gene and a nucleic acid from a wild-type gene corresponding to the mutant-type gene; a combination of a nucleic acid after artificial introduction of a mutation and a nucleic acid before artificial introduction of the mutation; a combination of a nucleic acid treated with bisulfite and a methylated nucleic acid before treatment with bisulfite; a combination of a nucleic acid after substitution, deletion or insertion occurs and a nucleic acid before the mutation occurs; and a combination of nucleic acids obtained from a living body, tissue, cell or the like before and after administration of a drug. In the present invention, depending on which nucleic acid of the above-mentioned combination is set as the target nucleic acid, the target nucleic acid and the non-target nucleic acid in the combination may be replaced by each other.

Hereinafter, the present invention will be explained in detail.

### (1) Selective cleavage method of non-target nucleic acid of the present invention

The present invention provides a method of selectively cleaving only a non-target nucleic acid in a sample containing a target nucleic acid and the non-target nucleic acid having a region of a nucleotide sequence homologous to the target nucleic acid. As used herein, "the non-target nucleic acid having a region of a nucleotide sequence homologous to the target nucleic acid" means that the nucleotide sequence of the non-target nucleic acid contains a nucleotide sequence homologous to at least a part of the nucleotide sequence of the target nucleic acid. Examples of the non-target nucleic acid include a non-target nucleic acid having a nucleotide sequence which differs from the nucleotide sequence of the target nucleic acid by substitution of one or more bases, a non-target nucleic acid having a nucleotide sequence which differs from the nucleotide sequence of the target nucleic acid by insertion of one or more bases, and a non-target nucleic acid having a nucleotide sequence which differs from the nucleotide sequence of the target nucleic acid by deletion of one or more bases. The number of the substituted, inserted, or deleted bases in the homologous sequence is not particularly limited. However, according to the present invention, the target nucleic acid and the non-target nucleic acid are distinguishable by the presence of at least one different base in their nucleotide sequences. As the number of different bases increases, the accuracy of discrimination between the target nucleic acid and the non-target nucleic acid is improved.

In the first aspect of the present invention, the non-target nucleic acid is selectively cleaved in a mixture of the target nucleic acid and the non-target nucleic acid having a region of a nucleotide sequence homologous to the target nucleic acid. The above-mentioned method uses an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid (hereinafter, sometimes, referred to as a suppressive oligonucleotide), and a polypeptide having a mismatch endonuclease activity. The number of the at least one mismatch formed when the suppressive oligonucleotide is hybridized with the non-target nucleic acid is not particularly limited as long as the selective cleavage of the non-target nucleic acid occurs, and examples thereof include 1 to 7, 1 to 5, or 1 to 3 mismatches depending on the length of the suppressive oligonucleotide. When the number of the at least one mismatch is expressed as a percentage in the length of the suppressive oligonucleotide, for example, the at least one mismatch accounts for 1-20%, 3-15%, or 4-8% of the length of the suppressive oligonucleotide. As an example of this aspect, a combination of the target nucleic acid and the non-target nucleic acid which differ by only one base in their nucleotide sequences is explained. When the suppressive oligonucleotide is hybridized with the target nucleic acid, a mismatch is formed between the suppressive oligonucleotide and the base which differs between the target nucleic acid and the non-target nucleic acid (hereinafter, sometimes, referred to as a first mismatch), while another mismatch is formed (hereinafter, sometimes, referred to as a second mismatch).

The base pair of the first mismatch relates to the base differing between the target nucleic acid and the non-target nucleic acid, regardless of whether the mismatched base pair is recognized and cleaved by the co-existing polypeptide having a mismatch endonuclease activity.

The second mismatch is a mismatched base pair which is recognized and cleaved by the co-existing polypeptide having a mismatch endonuclease activity. For example, when a polypeptide having a mismatch endonuclease activity to recognize and cleave guanine base-guanine base, guanine base-thymine base, or thymine base-thymine base is used, a suppressive oligonucleotide is designed to form a mismatch selected from the above-mentioned mismatched base pairs.

When the suppressive oligonucleotide is hybridized with the non-target nucleic acid, the second mismatch is formed, but the first mismatch is not formed.

Designing and using the suppressive oligonucleotide having the above-mentioned properties enable selective cleavage of a non-target nucleic acid by a polypeptide having a mismatch endonuclease activity. The present invention is not limited to use of the suppressive oligonucleotide capable of forming one or two mismatches as mentioned above. As long as selective cleavage of the desired nucleic acid occurs, a suppressive oligonucleotide capable of forming 3 or more mismatches may be designed and used. In such a case, at least one mismatch formed in a target nucleic acid and a non-target nucleic acid may be the second mismatch, and the other mismatches may or may not be recognized and cleaved by a polypeptide having a mismatch endonuclease activity. The 3 or more mismatches are preferably recognized and cleaved by a polypeptide having a mismatch endonuclease activity.

The suppressive oligonucleotide used in the method of the present invention is composed of DNAs, but a part of the DNAs may be replaced by a nucleotide analog or RNA. In other words, the suppressive oligonucleotide is not particularly limited as long as the suppressive oligonucleotide has a structure that forms a double-stranded nucleic acid having at least one mismatch when hybridized with a non-target nucleic acid and the mismatch is recognized and cleaved by a co-existing polypeptide having a mismatch endonuclease activity.

For example, when a difference of one base between two nucleic acids is distinguished by using a polypeptide having a mismatch endonuclease activity, an oligonucleotide is usually designed to form a mismatch with one of the two nucleic acids which may be originally recognized by the polypeptide having a mismatch endonuclease activity, and then used.

However, the mismatch originally recognized by the polypeptide having a mismatch endonuclease activity may not be formed depending on the base to be distinguished. In such a case, like the method of the present invention, a mismatch is made to be formed within a region which does not contain the base to be distinguished in the nucleic acid, and the mismatch thus formed is combined with formation/non-formation of a mismatch based on the above-mentioned different base, which allows for selective cleavage of one of the two nucleic acids. In other words, the present invention uses an oligonucleotide of such a nucleotide sequence that at least one mismatch originally recognized by a polypeptide having a mismatch endonuclease activity is formed when the oligonucleotide is hybridized with one of two nucleic acids which differ by at least one base in their nucleotide sequence, while the above-mentioned at least one mismatch as well as at least one mismatch based on the different base in the nucleotide sequences of the two nucleic acids are formed when the oligonucleotide is hybridized with the other of the two nucleic acids. When this oligonucleotide (suppressive oligonucleotide) and a polypeptide having a mismatch endonuclease activity are used in combination, a double-stranded nucleic acid in which one mismatch is formed is cleaved by the polypeptide, but such cleavage does not occur in a double-stranded nucleic acid in which two or more mismatches are formed.

Thus, another aspect of the method of the present invention provides a method characterized in that the non-target nucleic acid has a nucleotide sequence differing from the nucleotide sequence of the target nucleic acid by base substitution, and the sample is brought into contact with an oligonucleotide which forms at least one mismatch when the oligonucleotide is hybridized with the non-target nucleic acid and forms a mismatch corresponding to the at least one mismatch and at least another mismatch when the oligonucleotide is hybridized with the target nucleic acid, and a polypeptide having a mismatch endonuclease activity. In this aspect, the oligonucleotide includes an oligonucleotide which forms one mismatch (the second mismatch) when hybridized with the non-target nucleic acid or a complementary strand thereof and forms a mismatch corresponding to the above-mentioned mismatch and another mismatch (the first mismatch) when hybridized with the target nucleic acid or a complementary strand thereof.

For example, when a base at a base mutation site (for example, SNP) in a mutant-type gene cannot form a mismatch originally recognized by a polypeptide having a mismatch endonuclease activity, a suppressive oligonucleotide can be designed to have a nucleotide sequence that allows a mismatch at the SNP site in the mutant-type gene which is the target nucleic acid as well as another mismatch near the SNP site to be formed. The another mismatch is formed with a base unrelated to the SNP, that is, a base common to both the mutant-type gene and the wild-type gene, and can be recognized and cleaved by a polypeptide having a mismatch endonuclease activity. A base in the suppressive oligonucleotide corresponding to the base at the SNP site pairs with a base in the wild-type gene correctly. A double-stranded nucleic acid formed when the suppressive oligonucleotide is hybridized with the nucleic acid of the wild-type gene comprises one mismatch that can be cleaved by a polypeptide having a mismatch endonuclease activity, and thus the nucleic acid of the wild-type gene is selectively cleaved. On the other hand, the suppressive oligonucleotide cannot be stably hybridized with the nucleic acid of the mutant-type gene because two mismatches are formed, and further, cleavage by the polypeptide is inhibited. Thus, the method of the present invention is characterized in that selective cleavage of a wild-type gene by a polypeptide having a mismatch endonuclease activity is performed at a different position from a base mutation site in a co-existing mutant-type gene.

A further aspect of the method of the present invention provides a method characterized in that the non-target nucleic acid has a nucleotide sequence differing from the nucleotide sequence of the target nucleic acid by base insertion, and the sample is brought into contact with an oligonucleotide which forms at least one mismatch when the oligonucleotide is hybridized with a region containing the insertion in the nucleotide sequence of the non-target nucleic acid, and a polypeptide having a mismatch endonuclease activity. In this aspect, the region which the oligonucleotide (suppressive oligonucleotide) is hybridized with is preferably selected from regions which are not present in the target nucleic acid and are present in the non-target nucleic acid. Alternatively, the region which the oligonucleotide (suppressive oligonucleotide) is hybridized with may be a region extending over a region which is not present in the target nucleic acid and a region which is present in the target nucleic acid.

The suppressive oligonucleotide is designed to form at least one mismatch which is recognized and cleaved by the polypeptide having a mismatch endonuclease activity when the suppressive oligonucleotide is hybridized with the non-target nucleic acid. Since the oligonucleotide thus designed forms more mismatches with the target nucleic acid that does not have a nucleotide sequence inserted into the non-target nucleic acid, the formed double-stranded nucleic acid is destabilized, or the oligonucleotide cannot be hybridized with the target nucleotide. If further mismatches are made to be formed, the positions of the mismatches are not limited.

A further aspect of the method of the present invention provides a method characterized in that the non-target nucleic acid has a nucleotide sequence differing from the nucleotide sequence of the target nucleic acid by base deletion, and the sample is brought into contact with an oligonucleotide which forms at least one mismatch when the oligonucleotide is hybridized with a region containing the deleted site in the non-target nucleic acid, and a polypeptide having a mismatch endonuclease activity. In this aspect, the region which the oligonucleotide (suppressive oligonucleotide) is hybridized with is preferably selected from regions containing the deleted site in the non-target nucleic acid. The suppressive oligonucleotide is designed to form at least one mismatch which is recognized and cleaved by the polypeptide having a mismatch endonuclease activity when the suppressive oligonucleotide is hybridized with the non-target nucleic acid. Since the oligonucleotide thus designed forms more mismatches with the target nucleic acid that has a nucleotide sequence deleted in the non-target nucleic acid, the formed double-stranded nucleic acid is destabilized, or the oligonucleotide cannot be hybridized with the target nucleotide. If further mismatches are made to be formed, the positions of the mismatches are not limited.

The length of the suppressive oligonucleotide used in the method of the present invention is preferably a length of 7 bases or more, and examples thereof include, but not particularly limited to, a length within a range of 7-40 bases, preferably 10-30 bases, and more preferably 11-25 bases. The nucleotide sequence of the suppressive oligonucleotide is designed to form at least one mismatch when hybridized with the non-target nucleic acid and form more mismatches when hybridized with the target nucleic acid than when the oligonucleotide is hybridized with the non-target nucleic acid. At this time, selected is such a nucleotide sequence that when hybridized with the target nucleic acid (for example, a nucleic acid from a mutant-type gene) or a complementary strand thereof, a mismatch is formed with a base differing between the target nucleic acid and the non-target nucleic acid and at least another mismatched base pair is formed preferably up to 5 bases away from, more preferably up to 2 bases away from or next to the position of the different base in the direction of 5'-end side or 3'-end side. In other words, a nucleotide sequence that allows two mismatches to be located at an interval of not more than 5 bases is preferably used. In addition, the oligonucleotide is preferably designed to form both the mismatches in a region near the central base of the oligonucleotide. Further, when the oligonucleotide of this nucleotide sequence is hybridized with the non-target nucleic acid (for example, a nucleic acid from a wild-type gene), the oligonucleotide does not form a mismatch with a base differing between the target nucleic acid and the non-target nucleic acid. The mismatch formed with a base differing between the target nucleic acid and the non-target nucleic acid when the oligonucleotide is hybridized with the target nucleic acid or a complementary strand thereof may or may not be recognized and cleaved by a polypeptide having a mismatch endonuclease activity. When the oligonucleotide is hybridized with the target nucleic acid or the non-target nucleic acid or a complementary strand thereof, at least one of mismatches formed with bases other than the base differing between the target nucleic acid and the non-target nucleic acid is a mismatch recognized and cleaved by a polypeptide having a mismatch endonuclease activity. Such design of the suppressive oligonucleotide allows the method of the present invention to detect mutations in a great wide range of targets as compared with conventional techniques using restriction enzymes and the like. The 3'-end of the oligonucleotide may be modified so as to inhibit an extension reaction from the oligonucleotide by a DNA polymerase, to which the present invention is not particularly limited. Examples of the modification include amination.

In the method of the present invention, the suppressive oligonucleotide is used in combination with a polypeptide having a mismatch endonuclease activity which is optimal to recognition and cleavage of a mismatched base pair. Preferable examples of the polypeptide include, but not limited to, polypeptide PF0012 from Pyrococcus furiosus (WO2014/142261) and a mutant of the peptide (sometimes, collectively referred to as NucS protein). Homologs of the above-mentioned polypeptide which are derived from Pyrococcus abyssi (GenBank Accession No. Q9V2E8), Thermococcus barophilus (RsfSeq ID: YP_004072075), and Methanocaldococcus jannaschii (RsfSeq ID: NP247194), and their mutants may be also preferably used in the method of the present invention.

In a preferable aspect of the present invention, for nucleic acid amplification or detection which is performed simultaneously with the method of the present invention, a polypeptide having a mismatch endonuclease activity derived from a heat-resistant microorganism is preferably used. Preferable examples of the polypeptide include, but not limited to, the polypeptides retaining the activity even during thermal cycles such as PCR, and the polypeptides that are not inactivated at 50°C or more, preferably 70°C or more, more preferably 90°C or more.

The method of the present invention can be performed in the presence of an acidic high molecular substance. The acidic high molecular substance has been found to have effect of controlling the mismatch recognition and cleavage activity of the polypeptide by coexistence of the acidic high molecular substance. The acidic high molecular substance exerts more effect in a sample containing a small amount of nucleic acid. Preferable examples of the acidic high molecular substance include polyanions. Preferable examples of the acidic high molecular substance also include acidic polysaccharides having a sugar backbone and acidic polysaccharides having a liner carbon chain. As the acidic polysaccharides, one or more substances selected from the group consisting of fucose sulfate-containing polysaccharide, dextran sulfate, carrageenan, heparin, rhamnan sulfate, dermatan sulfate (chondroitin sulfate B), heparan sulfate, hyaluronic acid, alginic acid, pectin, polyglutamic acid, polyacrylic acid, polyvinyl sulfate, polystyrene sulfate, and their salts, and different nucleic acids from a target nucleic acid and a non-target nucleic acid can be used.

The method of the present invention may be performed further in combination with use of a proliferating cell nuclear antigen (PCNA).

### (2) Selective amplification method of target nucleic acid of the present invention

The selective amplification method of nucleic acid of the present invention is a method of selectively amplifying one (a target nucleic acid) of two nucleic acids (the target nucleic acid and a non-target nucleic acid) having nucleotide sequence regions similar to each other in a sample containing the two nucleic acids, which is characterized by comprising (1) a step of cleaving the non-target nucleic acid in the sample by the selective cleavage method of non-target nucleic acid of the present invention, and (2) a step of amplifying the target nucleic acid. The above-mentioned two steps may be performed separately and sequentially, or may be performed simultaneously. In the latter case, the cleavage of the non-target nucleic acid may occur during the nucleic acid amplification reaction.

In the selective amplification method of target nucleic acid of the present invention, the non-target nucleic acid is selectively cleaved by a polypeptide having a mismatch endonuclease activity, and the abundance ratio of the non-target nucleic acid in decreased, and as a result, the target nucleic acid is selectively amplified. In the step of nucleic acid amplification, a known nucleic acid amplification method can be used, and a nucleic acid amplification method which produces a condition allowing the suppressive oligonucleotide to be hybridized with the non-target nucleic acid is preferably used. More preferably, a PCR method can be used in the method of the present invention, but the present invention is not limited to use of the PCR method.

In the present invention, the nucleic acid amplification can be performed in the presence of an acidic high molecular substance. When both the above-mentioned steps (1) and (2) are simultaneously performed, improved efficiency of the nucleic acid amplification and improved efficiency of recognition and cleavage of the non-target nucleic acid by the polypeptide having a mismatch endonuclease activity can be attained at the same time by the effect of the acidic high molecular substance. The acidic high molecular substance exerts more effect in a sample containing a small amount of nucleic acid. Preferable examples of the acidic high molecular substance include the substances mentioned above in explanation of the selective cleavage method of non-target nucleic acid of the present invention.

Further, the method of the present invention may be performed in combination with use of a proliferating cell nuclear antigen (PCNA).

### (3) Selective detection method of target nucleic acid of the present invention

The selective detection method of target nucleic acid of the present invention is characterized by comprising (1) a step of selectively amplifying the target nucleic acid by the selective amplification method of target nucleic acid of the present invention, and (2) a step of detecting the target nucleic acid simultaneously with or after the above step (1). When the detection method is applied to a sample containing a large amount of a non-target nucleic acid and a very small amount of a target nucleic acid, the abundance ratio of the non-target nucleic acid and the target nucleic acid in the starting sample and the abundance ratio in the detection step are reversed. In other words, the target nucleic acid can be detected with high sensitivity by amplifying the target nucleic acid while suppressing the amplification of the non-target nucleic. In the detection method of the present invention, step (2) may be performed after step (1) or both step (1) and step (2) may be performed simultaneously. A detection method in step (2) may be a method for detecting or quantifying an amplified product, or a method for detecting an amplified product specifically to the nucleotide sequence of the amplified product. For example, for end point detection, a direct sequencing method or a high resolution melting curve analysis (HRM) method using intercalator can be used. For real-time detection, a cycling probe method using a sequence-specific probe (for example, WO2003/074696) or a TaqMan (registered trademark) assay method (for example, WO92/02638) can be used. More preferably, detection by the cycling probe method that can be combined with a PCR method is used.

Primer pairs used in the PCR method may be designed to allow a region in the target nucleic acid and the non-target nucleic acid containing a nucleotide sequence differing between the target nucleic acid and the non-target nucleic acid (i.e., a region which the suppressive oligonucleotide can be hybridized with) to be amplified. The design and synthesis of the primers can be performed by known methods.

When the detection is performed by the cycling probe method, the detection system may contain, but not limited to, a polypeptide having a ribonuclease H activity. The polypeptide having a ribonuclease H activity is preferably heat-resistant. Preferable examples of the polypeptide include, but not limited to, ribonuclease H derived from Bacillus caldotenax, from Pyrococcus furiosus, from Pyrococcus horikoshii, from Archaeoglobus fulgidus, from Thermococcus litoralis, from Thermococcus celer, from Thermotoga maritima, and from Archaeoglobus profundus.

The cycling probe method can detect the target nucleic acid as distinguished from the non-target nucleic acid. The probes (chimeric oligonucleotide probes) usable for such detection can be designed by a known method, for example, a method disclosed in WO2012/014988. Preferable examples of the chimeric oligonucleotide probes include those containing a RNA part sandwiched between two parts in which one of the two parts is labelled with a fluorescent substance and the other is labelled with a substance (quencher) that quenches the fluorescence produced from the fluorescent substance. Preferable examples of a combination of the substances include 6-FAM (6-carboxyfluorescein) and DABCYL (4-dimethylaminoazobenzene-4'-sulfone); ROX (6-carboxy-X-rhodamine) and DABCYL; 6-FAM and Eclipse (manufactured by Epoch Biosciences); ROX and Eclipse; TET (tetrachlorofluorescein) and DABCYL; and TET and Eclipse.

The suppressive oligonucleotide used in the selective detection method of target nucleic acid of the present invention is preferably designed to have the properties of the suppressive oligonucleotide as explained for the selective cleavage method of non-target nucleic acid of the present invention, and not to be recognized and cleaved by the co-existing polypeptide having a mismatch endonuclease activity when hybridized with the oligonucleotide probe used in the detection method of target nucleic acid.

In the detection method of the present invention using PCR, the method may be combined with amplification and detection of a positive control nucleic acid. The positive control nucleic acid is preferably a nucleic acid of a different gene originally existing in the sample (for example, a housekeeping gene or the like) from a target gene to be detected. When a gene originally existing in the sample is used as the positive control nucleic acid, a pair of primers for amplifying any region in the gene and a probe for detection are used in combination. An artificial nucleic acid may be also prepared and added to the sample previously. For example, the artificial nucleic acid may be a nucleic acid of the same region as the amplified regions of the target nucleic acid and the non-target nucleic acid, or a nucleic acid of a different region from the amplified regions of the target nucleic acid and the non-target nucleic acid. When a known amount of the artificial nucleic acid is added as the positive control nucleic acid to the sample previously, the same primer pair is used if the artificial nucleic acid can be amplified by using the same primers as used for the amplified regions of the target nucleic acid and the non-target nucleic acid, and a primer pair for amplifying a region of the artificial nucleic acid is used if the artificial nucleic acid is a different region from the amplified regions of the target nucleic acid and the non-target nucleic acid. As the probe for detecting the positive control nucleic acid, a probe capable of selectively detecting the positive control nucleic acid is used. Performing the detection of the target nucleic acid of the present invention and the detection of the positive control nucleic acid at the same time allows for confirmation of the presence or absence of an abnormality in amplification by the detection system of the present invention and semiquantitative analysis of the initial amount of the target nucleic acid based on comparison of amplification curves.

The detection method of the present invention can be performed in the presence of an acidic high molecular substance. The acidic high molecular substance exerts more effect in a sample containing a small amount of nucleic acid. Preferable examples of the acidic high molecular substance include the substances mentioned above in explanation of the selective cleavage method of non-target nucleic acid of the present invention. Further, the method of the present invention may be performed in combination with use of a proliferating cell nuclear antigen (PCNA).

Regarding a nucleic acid having a specific nucleotide sequence, for example, a nucleic acid corresponding to a gene wherein a mutation in the gene is known to be present, the detection method of the present invention can distinctively detect the wild-type gene and the mutant-type gene. When the detection method of the present invention is performed in which a nucleic acid having a nucleotide sequence of a mutant-type gene is set as the target nucleic acid and a nucleic acid having a nucleotide sequence of a wild-type gene is set as the non-target nucleic acid, a small number of a mutant allele can be detected in the presence of an excessively large amount of the normal allele (i.e., a DNA having the wild-type nucleotide sequence). For example, the method of the present invention is useful for detection of a circulating tumor DNA, or detection of a small amount of a fetal DNA sequence contained in the mother's blood. Examples of the mutation include microdeletion and point mutation.

Preferred examples of the nucleic acid having a specific nucleotide sequence include, but not limited to, nucleic acids containing at least one single nucleotide polymorphism selected from the group consisting of a single nucleotide polymorphism mutation used as a tumor marker, a single nucleotide polymorphism mutation correlating with a therapeutic effect of an agent for the treatment of cancer, and a single nucleotide polymorphism mutation known to correlate with canceration of cells. Examples of SNPs include those frequently found in tumor cells, and those known to correlate with a therapeutic effect of an agent for the treatment of cancer or carcinogenesis. Examples of such the acquired gene mutations include mutations of K-ras genes, B-raf genes, and epidermal growth factor receptor (EGFR) genes. Somatic mutations in the K-ras gene are frequently found in colorectal cancer, lung adenocarcinoma, thyroid cancer, and the like. Somatic mutations in the B-raf gene are frequently found in colorectal cancer, malignant melanoma, papillary thyroid cancer, non-small cell lung cancer, lung adenocarcinoma, and the like. Somatic mutations in the EGFR gene are frequently found in various solid tumors. It is known that the treatment of a cancer with an EGFR inhibitor such as gefitinib or erlotinib is likely to be effective when the EGFR gene in the cancer tissue has a specific single nucleotide polymorphism mutation. In contrast, it is known that a cancer is likely to be resistant to an EGFR inhibitor when the K-ras gene in the cancer tissue has a single nucleotide polymorphism mutation.

The detection method of the present invention may be also used for methylation analysis. For example, a methylated DNA extracted from a sample from an organism is set as a wild-type gene, and a DNA obtained after treatment of a composition containing the wild-type gene with bisulfate is set as a mutant-type gene. According to the detection method of the present invention, a small number of a methylated allele can be detected in the presence of an excessively large amount of a non-methylated allele, or a small number of a non-methylated allele can be detected in the presence of an excessively large amount of a methylated allele.

As the treatment with bisulfite, a known bisulfite method, which is used for detection of a methylated DNA can be used. By the treatment, non-methylated cytosine is changed into uracil, whereas methylated cytosine is not changed. When a reaction solution treated with bisulfite is subjected to amplification by PCR, uracil is changed into thymine and methylated cytosine is changed into cytosine. In other words, detection of a small number of a methylated allele in the presence of an excessively large amount of a non-methylated allele at a specific site, and detection of a small number of a non-methylated allele in the presence of an excessively large amount of a methylated allele respectively correspond to examination of the presence of cytosine in the presence of an excessively large amount of thymine, and examination of the presence of thymine in the presence of an excessively large amount of cytosine. When amplification of an excessively large amount of DNA containing thymine or cytosine is suppressed, the presence of a small number of a methylated allele or non-methylated allele is easily examined.

In the detection method of the present invention, amplification of a wild-type gene can be inhibited and a rare mutant-type gene can be selectively amplified and thus easily detected by adding the polypeptide having a mismatch endonuclease activity and the suppressive oligonucleotide that forms a mismatch with the wild-type gene to a reaction mixture. Further, as described later in Examples, the suppressive oligonucleotide capable of being recognized and cleaved by the polypeptide having a mismatch endonuclease activity can be widely designed regardless of the kind of a base at the base mutation site, by artificially designing a mismatch at an original base mutation site and at least another mismatch at a different site from the original base mutation site.

### (4) Composition of the present invention

Examples of a composition for the selective amplification method and/or selective detection method of target nucleic acid of the present invention include a composition containing (a) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid; (b) at least one pair of oligonucleotide primers; (c) a polypeptide having a mismatch endonuclease activity or a mutant thereof; and (d) a polypeptide having a DNA polymerase activity.

The composition may contain an acidic high molecular substance. Preferable examples of the acidic high molecular substance include the substances mentioned above in explanation of the selective cleavage method of non-target nucleic acid of the present invention.

The composition may further contain a proliferating cell nuclear antigen (PCNA).

The composition used for the selective detection method of target nucleic acid of the present invention wherein the detection of a target nucleic acid is performed by PCR may contain a polypeptide having a ribonuclease H activity or a mutant thereof, a cycling probe which is a chimeric oligonucleotide, or a TaqMan probe. Particularly, the polypeptide having a mismatch endonuclease activity, the polypeptide having a DNA polymerase activity, and the polypeptide having a ribonuclease H activity may be selected from a wild-type polypeptide or a mutant-type polypeptide. Preferable examples of the polypeptide having a mismatch endonuclease activity include, but not limited to, mutants of NucS protein from Pfu. Further, a pair of primers for detection of the positive control nucleic acid, and the chimeric oligonucleotide probe or the TaqMan probe may be used in combination.

### (5) Kit of the present invention

Examples of a kit for the selective amplification method and/or selective detection method of target nucleic acid of the present invention include a kit containing (a) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid; (b) at least one pair of oligonucleotide primers; (c) a polypeptide having a mismatch endonuclease activity or a mutant thereof; and (d) a polypeptide having a DNA polymerase activity or a mutant thereof.

The kit may further contain an acidic high molecular substance, and a solution of the acidic high molecular substance prepared at a suitable concentration can be preferably used. Preferable examples of the acidic high molecular substance include the substances mentioned above in explanation of the selective cleavage method of non-target nucleic acid of the present invention.

The kit may further contain a proliferating cell nuclear antigen (PCNA).

The kit used for the selective detection method of target nucleic acid of the present invention wherein the detection of a target nucleic acid is performed by PCR may contain a polypeptide having a ribonuclease H activity or a mutant thereof, a cycling probe which is a chimeric oligonucleotide, or a TaqMan probe. Particularly, the polypeptide having a mismatch endonuclease activity, the polypeptide having a DNA polymerase activity, and the polypeptide having a ribonuclease H activity may be selected from a wild-type polypeptide or a mutant-type polypeptide. Preferable examples of the polypeptide having a mismatch endonuclease activity include, but not limited to, mutants of NucS protein from Pfu. Further, a pair of primers for detection of the positive control nucleic acid, and the chimeric oligonucleotide probe or the TaqMan probe may be used in combination.

### (6) Selective cleavage method of non-target nucleic acid in the presence of acidic high molecular substance of the present invention

Another aspect of the cleavage method of a non-target nucleic acid of the present invention includes a method of selectively cleaving a non-target nucleic acid in a sample containing a target nucleic acid and the non-target nucleic acid having a region homologous to the target nucleic acid in the presence of an acidic high molecular substance, the method comprising a step of bringing the sample into contact with (i) the acidic high molecular substance; (ii) a polypeptide having a mismatch endonuclease activity; and (iii) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid.

In the method, preferable examples of the acidic high molecular substance, the polypeptide having a mismatch endonuclease activity, and the oligonucleotide include those mentioned above in explanation of the selective cleavage method of non-target nucleic acid of the present invention. The method of the present invention may be performed further in combination with use of a proliferating cell nuclear antigen (PCNA).

### Examples

Hereinafter, the present invention will be more specifically explained by way of Examples, which the present invention is not limited to.

### Example 1: Specific amplification of very small amount of commingling mutant-type gene EGFR T790M

### (1) Preparation of template for detection

From CDS sequence information of a human EGFR gene published in GenBank accession No. NC-000007, nucleotide sequences corresponding to primers EGFR_T790M_F and EGFR_T790M_R as described later, and a DNA containing a nucleotide sequence of a region between the nucleotide sequences of the primers were artificially synthesized. At this time, base C at nucleotide position 2369 in the EGFR gene was converted into T. By such base substitution, a codon for threonine (T) corresponding to amino acid position 790 is replaced by a codon for methionine (M) in the artificially synthesized DNA. The artificially synthesized gene thus obtained was designated EGFR codon790 DNA, and used as a mutant-type gene. Such a mutation is designated EGFR_T790M. In this case, a mismatched base pair can be designed to be formed at the site of T790M. As a DNA having a nucleotide sequence of a wild-type gene, a genomic DNA was prepared from human cell HL60.

In this Example, the target nucleic acid is EGFP_T790M DNA which has a mutation at EGFR codon position 790, and the non-target nucleic acid is HL60 genomic DNA.

### (2) Preparation of primers for amplification and suppressive oligonucleotide

Two primers having the nucleotide sequences shown in SEQ ID NOs: 1 and 2, EGFR_T790M_F and EGFR_T790M_R were prepared by a conventional method. Then, an oligonucleotide having the nucleotide sequence shown in SEQ ID NO: 3 which was hybridizable with a region containing the base at nucleotide position 2369 in the EGFR gene was prepared by a conventional method. To prevent an extension reaction from the 3' end of the oligonucleotide, a hydroxyl group at the 3' end was modified with an amino group. The oligonucleotide was designated suppressive oligonucleotide T790M_NucG-1. When the oligonucleotide was hybridized with the minus strand of the EGFR wild-type gene, a G-G mismatch was formed at the position of G complementary to C at nucleotide position 2369 (plus strand). On the other hand, when the oligonucleotide was hybridized with the minus strand of the mutant-type gene in which the base at nucleotide position 2369 was replaced by T, a G-A mismatch was formed at the position of A complementary to the T.

### (3) Design and preparation of probe for specific mutant-type gene detection

A chimeric oligonucleotide probe was synthesized as a probe for specifically detecting the EGFR codon790 DNA, wherein the oligonucleotide probe had a nucleotide sequence of a minus strand of a region extending one base in the 3' direction and 8 bases in the 5' direction from a position in the EGFR codon790 DNA corresponding to nucleotide number 2369 in the EGFR gene, and a DNA which was the 4th from the 5' end of the nucleotide sequence was replaced by an RNA. In addition, the 5' end of the oligonucleotide was bound to an Eclips quencher, and the 3' end of the oligonucleotide was bound to a FAM dye. The chimeric oligonucleotide probe thus prepared was designated T790M_detect-1. The nucleotide sequence of the T790M_detect-1 is shown in SEQ ID NO: 4.

Since the T790M_detect-1 is completely hybridized with the mutant-type EGFR gene in which the base at nucleotide position 2369 is replaced by T, the RNA part of the T790M_detect-1 is cleaved by coexistence of ribonuclease H. In contrast, when the T790M_detect-1 is hybridized with the wild-type EGFR gene, a mismatch is formed and thus the T790M_detect-1 does not undergo the cleavage by ribonuclease H.

### (4) Study of specific mutation detection method accompanied by suppressed amplification of wild-type gene

DNA samples were prepared by mixing the HL60 genomic DNA and the EGFR codon790 DNA as prepared in Example 1-(1) at ratios of 50,000 copies:500 copies (= 100:1), 50,000 copies:50 copies (= 1000:1), and 50,000 copies:5 copies = 10,000:1). The DNA amount of 50,000 copies of the HL60 genomic DNA is about 185 ng. As a control, a sample containing only the HL60 genomic DNA was prepared. These samples were used as templates.

A polypeptide having a mismatched nucleotide-specific double strand cleavage activity derived from Pyrococcus furiosus (hereinafter, referred to as NucS protein) which was used as the polypeptide having a mismatch endonuclease activity was a mutant of polypeptide PF0012 in which W77F was introduced, prepared by a method as described in Preparation Examples 1 to 3 and Example 1 in WO2014/142261.

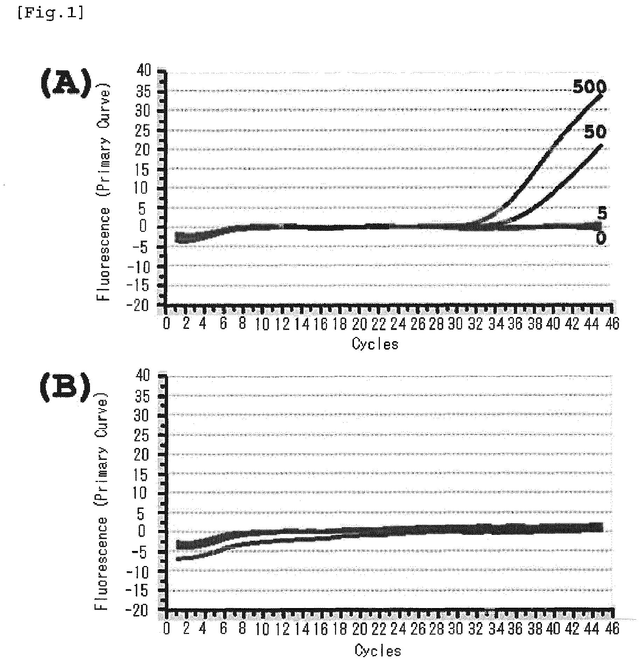

Detection of a specific mutation accompanied by suppressed amplification of the wild-type gene was performed as described below. A final volume of 25 µl of a reaction mixture containing the above-mentioned sample, 112 nM NucS protein, 0.2 µM T790M_NucG-1, a pair of each 0.2 µM EGFR_T790M primers, and 0.2 µM T790M_detect-1 was prepared using CycleavePCR reaction mix (manufactured by TAKARA BIO INC.). Real-time PCR detection was performed by thermal cycler TP900 (manufactured by TAKARA BIO INC.). The PCR was performed under the reaction conditions of initial denaturation treatment at 95°C for 10 seconds, and then 45 cycles of 95°C for 5 seconds, 55°C for 10 seconds and 72°C for 20 seconds. Fluorescent intensity was observed with time. At the same time, a reaction mixture not containing NucS protein was prepared as a control, and the same measurement was performed. Results are shown in Figure 1.

Specifically, Figure 1 shows results of the high sensitivity detection method for the T970M mutation in the EGFR gene. The vertical axis shows fluorescent intensity, and the horizontal axis shows the number of PCR cycles. Fluorescent curves of the copy numbers (0, 5, 50 and 500 copies) of the mutant-type gene are shown. As seen from Figure 1(B), when the reaction mixture did not contain NucS protein, an increase of a fluorescence value by the cleavage of the chimeric oligonucleotide probe T790M_detect-1 was not observed. This suggests that amplification of the DNA from the EGFR codon790 DNA was suppressed by the coexisting HL60 genomic DNA.

In contrast, as seen from Figure 1(A), when PCR was performed using the reaction mixture containing NucS protein, an increase of a fluorescence value was observed with time even in the sample containing 5 copies of the EGFR codon790 DNA relative to 50,000 copies of the HL60 genomic DNA. In other words, the DNA amplified from the EGFR codon790 DNA as a template was specifically detected by the T790M_detect-1. This result shows that the combination of NucS and the suppressive oligonucleotide suppresses the DNA amplification from the HL60 genomic DNA as a template, and thus enables the detection of the EGFR codon790 DNA.

### Example 2: Specific detection of EGFR gene having L858R mutation

### (1) Preparation of template for detection

From CDS sequence information of a human EGFR gene published in GenBank accession No. NC_000007, nucleotide sequences corresponding to primers EGFR_L858R_F and EGFR_L858R_R as described later, and a DNA containing a nucleotide sequence of a region between the nucleotide sequences of the primers were artificially synthesized. At this time, base T at nucleotide position 2573 in the EGFR gene was converted into G. By such base substitution, a codon for leucine (L) corresponding to amino acid position 858 is replaced by a codon for arginine (R) in the artificially synthesized DNA. The artificially synthesized gene thus obtained was designated EGFR codon858 DNA, and used as a mutant-type gene. Such a mutation is designated EGFR_L858R. In this case, a mismatched base pair cannot be designed to be formed at the site of L858R. As a wild-type gene, the human cell HL60 genomic DNA as prepared in Example 1 was used.

In this Example, the target nucleic acid is EGFR_L858R DNA which has a mutation at EGFR codon position 858, and the non-target nucleic acid is the HL60 genomic DNA.

### (2) Preparation of primers for amplification and suppressive oligonucleotide

Two primers having the nucleotide sequences shown in SEQ ID NOs: 5 and 6, EGFR_L858R_F and EGFR_L858R_R were prepared by a conventional method. Then, an oligonucleotide having the nucleotide sequence shown in SEQ ID NO: 7 which was hybridizable with a region containing nucleotide position 2573 in the EGFR gene was prepared. To prevent an extension reaction from the 3' end of the oligonucleotide, a hydroxyl group at the 3' end was modified with an amino group. The oligonucleotide was designated suppressive oligonucleotide EGFR_L858R_sup_p3. The nucleotide sequence of the EGFR_L858R_sup_p3 is shown in SEQ ID NO: 7. When the oligonucleotide was hybridized with the plus strand of the EGFR wild-type gene, a G-T mismatch was formed at the position of G at nucleotide position 2575. On the other hand, when the oligonucleotide was hybridized with the plus strand of the mutant-type gene in which the base at nucleotide position 2573 was replaced by G, a G-A mismatch was formed at the position of G, and in addition, a G-T mismatch was also formed at the position of G which was the second (nucleotide position 2575) in the 3' direction from the replacement site.

### (3) Design and preparation of probe for specific mutant-type gene detection

A chimeric oligonucleotide probe was synthesized as a probe for specifically detecting the EGFR codon858 DNA, wherein the oligonucleotide probe had a nucleotide sequence of a complementary strand to a region extending one base in the 3' direction and 9 bases in the 5' direction from a position in the EGFR codon858 DNA corresponding to nucleotide number 2573 in the EGFR gene, and a DNA which was the third from the 5' end of the nucleotide sequence was replaced by an RNA. In addition, the 5' end of the oligonucleotide was bound to an Eclips quencher, and the 3' end of the oligonucleotide was bound to a FAM dye. The chimeric oligonucleotide probe thus prepared was designated EGFR_L858R_P2. The nucleotide sequence of the EGFR_L858R_P2 is shown in SEQ ID NO: 8.

Since the EGFR_L858R_P2 is completely hybridized with the mutant-type EGFR gene in which base T at nucleotide position 2573 is replaced by G, the RNA part of the EGFR_L858R_P2 is cleaved by coexistence of ribonuclease H. In contrast, when the EGFR_L858R_P2 is hybridized with the wild-type EGFR gene, a mismatch is formed and thus the EGFR_L858R_P2 does not undergo the cleavage by ribonuclease H.

### (4) Study of specific mutation detection method accompanied by suppressed amplification of wild-type gene

DNA samples were prepared by mixing the HL60 genomic DNA and the EGFR codon858 DNA at ratios of 50,000 copies:500 copies (= 100:1), 50,000 copies:50 copies (= 1000:1), and 50,000 copies:5 copies = 10,000:1). As a control, a sample containing only the HL60 genomic DNA was prepared. The DNA amount of 50,000 copies of the HL60 genomic DNA is about 185 ng.

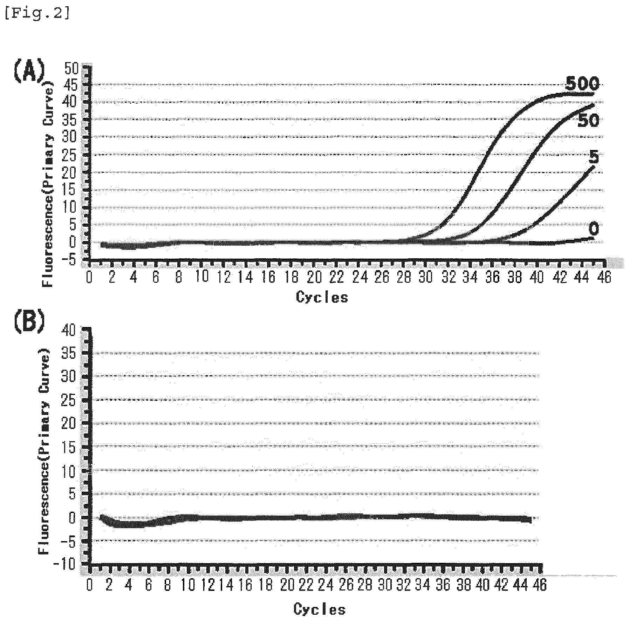

Specific mutation detection accompanied by suppressed amplification of the wild-type gene was performed as described below. A final volume of 25 µl of a reaction mixture containing the above-mentioned DNA sample, 112 nM NucS protein, 0.2 µM EGFR_L858R_sup_p3, a pair of each 0.2 µM EGFR_L858R primers, and 0.2 µM EGFR_L858R_sup_P2 was prepared using CycleavePCR reaction mix (manufactured by TAKARA BIO INC.). Real-time PCR detection was performed by thermal cycler TP900 (manufactured by TAKARA BIO INC.). The PCR was performed under the reaction conditions of initial denaturation treatment at 95°C for 10 seconds, and then 45 cycles of 95°C for 5 seconds, 55°C for 10 seconds and 72°C for 20 seconds. Fluorescent intensity was observed with time. At the same time, a group of reaction mixtures that did not contain NucS protein were prepared as a control, and the same measurement was performed. Results are shown in Figure 2.

Specifically, Figure 2 shows results of the detection method for the EGFR gene L858R mutation. The vertical axis shows fluorescent intensity, and the horizontal axis shows the number of PCR cycles. Fluorescent curves of the copy numbers (0, 5, 50 and 500 copies) of the mutant-type gene are shown. As seen from Figure 2(B), when the reaction mixture did not contain NucS protein, an increase of a fluorescence value by the cleavage of the chimeric oligonucleotide probe EGFR_L858R_P2 was not observed. Thus, it was shown that amplification of the DNA from the EGFR codon858 DNA was suppressed by the coexisting HL60 genomic DNA.

In contrast, as seen from Figure 2(A), when PCR was performed using the reaction mixture containing NucS protein, an increase of a fluorescence value, that is, cleavage of the chimeric oligonucleotide EGFR_L858R_P2 was observed even in the sample containing 5 copies of the mutant-type gene relative to 50,000 copies of the wild-type gene. In other words, it was shown that the EGFR_L858R mutant-type gene can be detected in the presence of an excessively large amount of the coexisting wild-type gene.

This result shows that a system for selectively cleaving one of two nucleic acids distinctively based on the existence or non-existence of the base substitution in the nucleic acids can be constructed by combining the suppressive oligonucleotide and the polypeptide having a mismatch endonuclease activity (NucS protein) as provided by the present invention, even when the nucleic acid contains the base substitution which cannot form a mismatch recognized by the polypeptide. Further, it was shown that amplification of a nucleic acid from a wild-type gene existing at a high abundance ratio is suppressed and a nucleic acid from a mutant-type gene is preferentially amplified by combining the above-mentioned combination with nucleic acid amplification reaction, and thereby the mutant-type gene can be detected with high sensitivity.

### Example 3: Investigation of detection method of target nucleic acid in the presence of acidic high molecular substance

### (1) Investigation of mutation detection method in the presence of acidic high molecular substance

A series of DNA samples were prepared as described in Example 2(4). In the same way, DNA samples were prepared by mixing the HL60 genomic DNA and the EGFR codon858 DNA at ratios of 5,000 copies:500 copies (= 10:1), 5,000 copies:50 copies (= 100:1), and 5,000 copies:5 copies = 1,000:1). As a control, a sample not containing the template DNA was prepared. The DNA amount of 5,000 copies of the HL60 genomic DNA is about 18.5 ng.

A final volume of 25 µl of a reaction mixture containing the above-mentioned DNA sample, 112 nM NucS protein, 0.2 µM EGFR_L858R_sup_p3, a pair of each 0.2 µM EGFR_L858R primers, 0.2 µM EGFR_L858R_sup_P2, and a final concentration of 56 ug/ml, 140 µg/ml or 168 µg/ml of sodium alginate was prepared using CycleavePCR reaction mix (manufactured by TAKARA BIO INC.). For comparison, a reaction mixture of the same composition except that it did not contain sodium alginate was prepared.

Real-time PCR detection was performed by thermal cycler TP900 (manufactured by TAKARA BIO INC.). The PCR was performed under the reaction conditions of initial denaturation treatment at 95°C for 10 seconds, and then 45 cycles of 95°C for 5 seconds, 55°C for 10 seconds and 72°C for 20 seconds. Fluorescent intensity was observed with time.

### (2) Analysis of effect of acidic high molecular substance

Results of the real-time PCR detection are shown in Figure 3. Specifically, Figure 3 shows results of effects of the acidic high molecular substance in the mutant-type gene detection method of the present invention. The vertical axis shows fluorescent intensity, and the horizontal axis shows the number of PCR cycles. Fluorescent curves of no template DNA (N.C.) and the copy numbers (5, 50 and 500 copies) of the mutant-type gene are shown. Figures 3(A-1), (B-1), (C-1) and (D-1) show results in the case where 50,000 copies of the HL60 genomic DNA were present, and Figures 3 (A-1), (B-2), (C-2) and (D-2) show results in the case where 5,000 copies of the HL60 genomic DNA were present. Figures 3(A), (B), (C) and (D) show effects in the presence of no sodium alginate, 56 µg/ml, 140 µg/ml and 168 µg/ml of sodium alginate respectively. It was found that sodium alginate at any concentration increased detection efficiency as compared with the absence of sodium alginate. Particularly, increased effects were shown in the case of a small amount of the template DNA (5,000 copies of the HL genomic DNA). Thus it was found that a mutant-type gene can be efficiently detected by combining use of an acidic high molecular substance with the detection method of the present invention even when the amount of DNA from a sample is small.

### Example 4: Effect of acidic high molecular substance on nucleic acid cleavage reaction by polypeptide having mismatch endonuclease activity

The effects of an acidic high molecular substance were further studied. A reaction mixture was prepared as described below. A final volume of 25 µl of a reaction mixture containing 5 copies (0.16 fg) of the EGFR codon790 DNA, 112 nM NucS protein, 0.2 µM T790M_MucG-1, a pair of each 0.2 µM EGFR_T790M primers, 0.2 µM T790M_detect-1, and a final concentration of 140 µg/ml of sodium alginate was prepared using CycleavePCR reaction mix (manufactured by TAKARA BIO INC.). This reaction mixture was designated Reaction mixture 4. Further, Reaction mixture 1 which was the same as Reaction mixture 4 except that it did not contain NucS protein and sodium alginate, Reaction mixture 2 which was the same as Reaction mixture 4 except that it did not contain NucS protein, and Reaction mixture 3 which was the same as Reaction mixture 4 except that it did not contain sodium alginate were prepared. Real-time PCR detection was performed under the conditions as described in Example 1.

As a result, amplification of the mutant-type gene was observed in Reaction mixture 1, Reaction mixture 2 and Reaction mixture 4, though these reaction mixtures contained very small copy number of the mutant-type gene. In contrast, the amplification was not observed in Reaction mixture 3.

It was suggested that unexpected hybridization could occur between the suppressive oligonucleotide and the mutant-type gene in Reaction mixture 3 because the wild-type gene was not present in the reaction mixture, and then could be cleaved by NucS protein. In contrast, it was suggested that in Reaction mixture 4, the acidic high molecular substance interacted with NucS protein and thereby the unexpected cleavage was suppressed.

Thus, it was found that an acidic high molecular substance has an effect of controlling the mismatch recognition and cleavage activity of NucS protien by interacting with the NucS protein.

### Example 5: Specific detection of EGFR_exon 19 deletion mutated gene

Detection of deletion mutation of exon 19 in the EGFR gene using the method of the present invention was studied. In this Example, EGFR_19_delE746-A750 DNA is the target nucleic acid and human genomic DNA (manufactured by TAKARA BIO INC.) is the non-target nucleic acid.

### (1) Preparation of template for detection

From CDS sequence information of human EGFR published in GenBank accession No. NC_000007, nucleotide sequences corresponding to primers EGFR_19_delE746-A750_F and EGFR_19_delE746-A750_R, and a DNA containing a nucleotide sequence of a region between the nucleotide sequences of the primers and deleting nucleotides from position 2236 to position 2250 were artificially synthesized. By such base deletion, amino acids from position 746 to position 750 in EGFR are deleted in the artificially synthesized DNA. The artificially synthesized gene thus obtained was designated EGFR_19_delE746-A750 DNA, and used as a mutant-type gene.

### (2) Preparation of primers for amplification and suppressive oligonucleotide

Two primers having the nucleotide sequences shown in SEQ ID NOs: 9 and 10, EGFR_19_delE746-A750_F and EGFR_19_delE746-A750_R were prepared by a conventional method. Then, an oligonucleotide having the nucleotide sequence shown in SEQ ID NO: 11 which was hybridizable with the deleted region in the EGFR gene was prepared. To prevent an extension reaction from the 3' end of the oligonucleotide, a hydroxyl group at the 3' end was modified with an amino group. The oligonucleotide was designated suppressive oligonucleotide EGFR_19 delE746-A750 oligo-7. When the oligonucleotide is hybridized with the plus strand of the EGFR wild-type gene, a G-C mismatch is formed at the position of G at nucleotide position 2245. On the other hand, the oligonucleotide is not hybridized with the plus strand of the deletion mutant-type gene.

### (3) Design and preparation of probe for specific mutant-type gene detection

A chimeric oligonucleotide probe was synthesized as a probe for specifically detecting the EGFR_19_delE746-A750 DNA, wherein the oligonucleotide probe had a nucleotide sequence of a complementary strand to a region in the DNA corresponding to nucleotide position 2228 to 2235 and nucleotide position 2251 to 2253 in the EGFR gene, and a DNA which was the 3rd from the 5' end of the nucleotide sequence was replaced by an RNA. In addition, the 5' end of the oligonucleotide was bound to an Eclips quencher, and the 3' end of the oligonucleotide was bound to a FAM dye. The chimeric oligonucleotide probe thus prepared was designated EGFR_2236-2250del. The nucleotide sequence of the EGFR_2236-2250del is shown in SEQ ID NO: 12.

Since the EGFR_2236-2250del is completely hybridized with the mutant-type EGFR gene in which the nucleotide sequence of nucleotide position 2236 to 2250 is deleted, the RNA part of the EGFR_2236-2250del is cleaved by coexistence of ribonuclease H. In contrast, the EGFR_2236-2250del is not hybridized with the wild-type EGFR gene, and thus the EGFR_2236-2250del does not undergo the cleavage by ribonuclease H.

### (4) Investigation of specific mutation detection method accompanied by suppressed amplification of wild-type gene

DNA samples were prepared by mixing the human genomic DNA and the EGFR_19_delE746-A750 DNA at ratios of 50,000 copies:500 copies (= 100:1), 50,000 copies:50 copies (= 1000:1), and 50,000 copies:5 copies = 10,000:1). As a control, the human genomic DNA was only used. The DNA amount of 50,000 copies of the human genomic DNA is about 185 ng.

Specific mutation detection accompanied by suppressed amplification of the wild-type gene was performed as described below. A final volume of 25 µl of a reaction mixture containing the above-mentioned DNA sample, 687.5 nM NucS protein, 0.2 µM EGFR_2236-2250del, a pair of 0.2 µM EGFR_19_delE746-A750 primers, 0.2 µM EGFR_19_delE746-A750 oligo-7, and a final concentration of 140 µg/ml of sodium alginate was prepared using CycleavePCR reaction mix. Real-time PCR detection was performed by thermal cycler TP900. The PCR was performed under the reaction conditions of initial denaturation treatment at 95°C for 10 seconds, and then 45 cycles of 95°C for 5 seconds, 55°C for 10 seconds and 72°C for 20 seconds. Fluorescent intensity was observed with time. At the same time, a group of reaction mixtures that did not contain NucS protein were prepared as a control, and the same measurement was performed. Results are shown in Figure 4.

Specifically, Figure 4 shows results of the detection method for the exon 19 deletion mutation in the EGFR gene. The vertical axis shows fluorescent intensity, and the horizontal axis shows the number of PCR cycles. Fluorescent curves of the copy numbers (0, 5, 50 and 500 copies) of the mutant-type gene are shown. When the reaction mixtures containing NucS protein was used for PCR, an increase of a fluorescence value, i.e. cleavage of the chimeric oligonucleotide EGFR_2236-2250del was observed even in the sample in which 5 copies of the mutant-type gene relative to 50,000 copies of the wild-type gene coexisted. In contrast, when the reaction mixture did not contain NucS protein, an increase of a fluorescence value by the cleavage of the chimeric oligonucleotide probe EGFR_2236-2250del was not observed. In other words, it was shown that amplification of the DNA from the EGFR_19_delE746-A750 DNA was suppressed because of the coexistence of the human genomic DNA.

This result shows that the desired mutant-type gene can be detected with high sensitivity even in the presence of an excessively large amount of the wild-type gene by combining the suppressive oligonucleotide and the polypeptide having a mismatch endonuclease activity (NucS protein) as provided by the present invention, without trouble even when the mutant-type gene contains a deletion-type genetic mutation.

### Example 6: Mutation analysis of k-ras gene codon 12 and codon 13

Detection of point mutations at codon 12 and codon 13 in the k-ras gene using the method of the present invention was studied. In this Example, a codon 12 point mutant and a codon 13 point mutant of the k-ras gene are the target nucleic acids, and the HL60 genomic DNA is the non-target nucleic acid.

### (1) Preparation of template for detection

From information of human K-ras gene published in GenBank accession No. NC_000012.12, primers k-rasG12_F and k-rasG12_R having the nucleotide sequences shown in SEQ ID NOs: 13 and 14 were chemically synthesized. Further, DNAs having a nucleotide sequence of a region between the nucleotide sequences of the primers in which the amino acid specified by codon 12 was changed from glycine to serine, from glycine to cysteine, from glycine to arginine, from glycine to aspartic acid, from glycine to valine, and from glycine to alanine by point mutation of codon 12 were artificially synthesized. These artificially synthesized DNAs were each inserted into a T-cloning site of T-Vector pMD20 (manufactured by TAKARA BIO INC.) by a conventional method. The plasmids thus prepared were designated k-rasG12S DNA, k-rasG12C DNA, k-rasG12R DNA, k-rasG12D DNA, k-rasG12V DNA, and k-rasG12A DNA, and used as mutant-type genes.

In the same way, DNAs having a nucleotide sequence of a region between the nucleotide sequences corresponding to the primer pair of k-rasG12_F and k-rasG12_R in which the amino acid specified by codon 13 was changed from glycine to serine, from glycine to cysteine, from glycine to arginine, from glycine to aspartic acid, and from glycine to alanine by point mutation of codon 13 were artificially synthesized. The artificially synthesized DNAs thus obtained were each inserted into a T-cloning site of T-Vector pMD20 by a conventional method. The plasmids thus prepared were designated k-rasG13S DNA, k-rasG13C DNA, k-rasG13R DNA, k-rasG13D DNA, and k-rasG13A DNA, and used as mutant-type genes.

### (2) Preparation of suppressive oligonucleotide

An oligonucleotide having the nucleotide sequence shown in SEQ ID NO: 15 which was hybridizable with a region containing codon 12 and codon 13 in the k-ras gene was prepared. To prevent an extension reaction from the 3' end of the oligonucleotide, a hydroxyl group at the 3' end was modified with an amino group. The oligonucleotide was designated suppressive oligonucleotide k-rasG12/13_S1. When the oligonucleotide is hybridized with the plus strand of the k-ras codon 12 wild-type gene having the nucleotide sequence shown in SEQ ID NO: 16, a G-G mismatch is formed at the position of G at nucleotide position 35. On the other hand, when the oligonucleotide is hybridized with the plus strand of the k-rasG12S DNA, a G-G mismatch is formed at the position of G at nucleotide position 35, and in addition, an A-C mismatch is formed at the position of A at nucleotide position 34. When the oligonucleotide is hybridized with the plus strand of the k-rasG12C DNA, a G-G mismatch is formed at the position of G at nucleotide position 35, and in addition, a T-C mismatch is formed at the position of T at nucleotide position 34. When the oligonucleotide is hybridized with the plus strand of the k-rasG12R DNA, a G-G mismatch is formed at the position of G at nucleotide position 35, and in addition, a C-C mismatch is formed at the position of C at nucleotide position 34. When the oligonucleotide is hybridized with the plus strand of the k-rasG12D DNA, an A-G mismatch is formed at the position of A at nucleotide position 35. When the oligonucleotide is hybridized with the plus strand of the k-rasG12V DNA, a T-G mismatch is formed at the position of T at nucleotide position 35. When the oligonucleotide is hybridized with the plus strand of the k-rasG12A DNA, no mismatch is formed.

When the suppressive oligonucleotide k-rasG12/13_S1 is hybridized with the plus strand of the k-ras codon 13 wild-type gene, a G-G mismatch is formed at the position of G at nucleotide position 35. On the other hand, when the oligonucleotide is hybridized with the plus strand of the k-rasG13S DNA, a G-G mismatch is formed at the position of G at nucleotide position 35, and in addition, an A-C mismatch is formed at the position of A at nucleotide position 37. When the oligonucleotide is hybridized with the plus strand of the k-rasG13C DNA, a G-G mismatch is formed at the position of G at nucleotide position 35, and in addition, a T-C mismatch is formed at the position of T at nucleotide position 37. When the oligonucleotide is hybridized with the plus strand of the k-rasG13R DNA, a G-G mismatch is formed at the position of G at nucleotide position 35, and in addition, a C-C mismatch is formed at the position of C at nucleotide position 37. When the oligonucleotide is hybridized with the plus strand of the k-rasG13D DNA, a G-G mismatch is formed at the position of G at nucleotide position 35, and in addition, an A-C mismatch is formed at the position of A at nucleotide position 38. When the oligonucleotide is hybridized with the plus strand of the k-rasG13A DNA, a G-G mismatch is formed at the position of G at nucleotide position 35, and in addition, a C-C mismatch is formed at the position of C at nucleotide position 38.

### (3) Confirmation of suppressed amplification of wild-type gene

A DNA sample was prepared by mixing the human genomic DNA and the k-rasG12C DNA at a ratio of 50,000 copies: 50 copies (= 1000:1). As a control, the human genomic DNA was only used. The DNA amount of 50,000 copies of the human genomic DNA is about 185 ng.

In the same way, a DNA sample was also prepared by mixing the human genomic DNA and the k-rasG13C DNA at a ratio of 50,000 copies:50 copies (= 1000:1).

A final volume of 25 µl of a reaction mixture containing the above-mentioned DNA sample, 1000 nM NucS protein, a pair of each 0.2 µM k-rasG12_F and k-rasG12_R primers, 0.2 µM k-rasG12/13_S1, and a final concentration of 140 µg/ml of sodium alginate was prepared. PCR detection was performed by thermal cycler TP900. The PCR was performed under the reaction conditions of initial denaturation treatment at 95°C for 10 seconds, and then 45 cycles of 95°C for 5 seconds, 55°C for 10 seconds and 72°C for 20 seconds. Amplified fragments thus obtained were purified by TaKaRA MiniBEST DNA Fragment Purification Kit ver. 4.0 (manufactured by TAKARA BIO INC.), and then subjected to direct sequencing using an oligonucleotide having the nucleotide sequence shown in SEQ ID NO: 13.

From results of the direct sequencing, mainly amplified products from the above-mentioned samples containing the k-rasG12C DNA were identified as amplified products from the k-rasG12C DNA. In addition, mainly amplified products from the samples containing the k-rasG13C DNA were also identified as amplified products from the k-rasG13C DNA. Thus it was found that amplification of a wild-type gene is suppressed and a mutant-type gene is preferentially amplified by the method of the present invention. In other words, it was shown that a mutant-type gene can be selectively concentrated by the method of present invention.

### Example 7: High sensitivity detection method in combination with acidic high molecular substance

The effects of an acidic high molecular substance were further studied on the model of the specific detection method of the EGFR gene having an L858R mutation as described in Example 2. In this Example, use of a salt of chondroitin sulfate B having a sugar chain backbone or polyacrylic acid having no sugar chain backbone as the acidic high molecular substance was studied.

### (1) Effect of acidic high molecular substance - 1

A final volume of 25 µl of a reaction mixture containing the above-mentioned DNA sample as prepared in Example 3(1), 125 nM NucS protein, 0.2 µM EGFR_L858R_sup_p3, a pair of each 0.2 µM EGFR_L858R primers, 0.2 µM EGFR_L858R_sup_P2, and a final concentration of 30 µg/ml, 40 µg/ml, 50 µg/ml or 60 µg/ml of sodium chondroitin sulfate B (manufactured by SIGMA-ALDRICH) was prepared using CycleavePCR reaction mix. For comparison, a reaction mixture of the same composition except that it did not contain sodium chondroitin sulfate B was also prepared.

Real-time PCR detection was performed under the same conditions as Example 3(1). As a result, as compared with the case where the reaction mixture did not contain sodium chondroitin sulfate B, a Ct value was clearly decreased when the reaction mixture contained sodium chondroitin sulfate B at any final concentration. Thus it was found that an acidic high molecular substance having a sugar chain backbone is useful in the method of the present invention.

### (2) Effect of acidic high molecular substance - 2

Next, use of polyacrylic acid 5000 (manufactured by Wako Pure Chemical Industries, Ltd.) as the acidic high molecular substance having no sugar chain backbone was studied. A final volume of 25 µl of a reaction mixture of the same composition as described in above (1) was prepared except that it contained a final concentration of 1 µg/ml, 2 µg/ml, 3 µg/ml, 4 µg/ml or 5 µg/ml of polyacrylic acid 5000 instead of sodium chondroitin sulfate B.

As a result of real-time PCR detection, as compared with the case where the reaction mixture did not contain polyacrylic acid, a Ct value was clearly decreased when the reaction mixture contained polyacrylic acid at any final concentration. Thus it was found that an acidic high molecular substance having no sugar chain backbone is useful in the method of the present invention.

### Example 8: Method of present invention in combination with amplification of positive control nucleic acid

A method for quantitating a target nucleic acid using the method of the present invention was studied. The EGFR_L858R was used as the target nucleic acid. Another region in the EGFR gene was used as a positive control nucleic acid. Specifically, primers EGFRL858R_IC2_F and EGFRL858R_IC2_R having the nucleotide sequences shown in SEQ ID NOs: 17 and 18 were synthesized by a conventional method. Further, a chimeric oligonucleotide probe was synthesized as a probe for detecting the positive control nucleic acid, wherein the oligonucleotide probe had the nucleotide sequence shown in SEQ ID NO: 19 and a DNA which was the 3rd from the 5' end of the nucleotide sequence was replaced by an RNA. In addition, the 5' end of the oligonucleotide was bound to an Eclips quencher, and the 3' end of the oligonucleotide was bound to a FAM dye. The chimeric oligonucleotide probe thus prepared was designated EGFRL858R_IC2_P2.

As a template DNA, the DNA samples as prepared in Example 2 were used. Reaction mixtures were prepared as described below. Specifically, a final volume of 25 µl of a reaction mixture containing the DNA sample, 125 nM NucS protein, 0.2 µM EGFR_L858R_sup_p3, a pair of each 0.2 µM EGFR_L858R primers, 0.2 µM EGFR_L858R_sup_P2, and a final concentration of 56 µg/ml of sodium alginate, and a pair of each 0.2 µM EGFRL858R_IC2 primers, and 0.2 µM EGFRL858R_IC2_P2 was prepared using CycleavePCR reaction mix. Real-time PCR detection was performed under the same conditions as described in Example 3.

The real-time PCR detection showed the same results as Example 3, regardless of amplification of the positive control nucleic acid performed at the same time. Thus it was found that errors between reaction mixtures can be corrected on the basis of the amplified amount of a positive control nucleic acid as an index. It was also found that the presence of a target nucleic acid in a sample can be quantitated by comparing the amplified amount of the amplified control nucleic acid and the amplified amount of the target nucleic acid in the sample. Thus, it was found that a mutant-type gene is efficiently detected by the detection method of the present invention even when the amount of DNA from a sample was small, and the mutant-type gene can be also quantitated by comparison with amplification of a positive control nucleic acid.

### Example 9: Method of present invention in combination with PCNA

Effects of PCNA in the method of the present invention were studied. The composition of a reaction mixture was the same as described in Example 8 except that the reaction mixture contained EGFRL858R_IC2_P3 as the chimeric oligonucleotide probe which had the nucleotide sequence shown in SEQ ID NO: 20 and in which the 3rd DNA from the 5' end of the nucleotide sequence was replaced by an RNA, 100 µg/ml of sodium chondroitin sulfate B, 8 µg/ml of a PufPCNA D143R mutant (PCNA13) prepared by a method as described in WO 2007/004654, and 375 nM of NucS protein. Real-time PCR detection was performed under the same conditions as described in Example 3.

As a result of the real-time PCR detection, a Ct value was clearly decreased regardless of amplification of the positive control nucleic acid performed at the same time when the reaction mixture contained PCNA, as compared with the case where the reaction mixture did not contain PCNA. Thus it was found that use of PCNA further improves the method of the present invention.

### Industrial Applicability

According to the high sensitivity mutation detection method of the present invention, amplification of a wild-type gene that is present in a large amount can be eliminated and a mutant-type gene that is present in a very small amount can be specifically amplified and detected by artificially allowing formation of a mismatched base pair to occur, regardless of the type of a mismatch that a polypeptide having a mismatch endonuclease activity can originally recognize. The method of the present invention is useful in broad fields including the fields of genetic technology, biology, medicine, and agriculture.

### Sequence Listing Free text

SEQ ID NO: 1: EGFR_T790M_F primer
SEQ ID NO: 2: EGFR_T790M_R primer
SEQ ID NO: 3: T790M_NucG-1. 3'-end is modified by amino linker (NH2).
SEQ ID NO: 4: T790M_detect-1. 5'-end is added Eclips and 3'-end is added FAM. nucleotide position 4 is RNA.
SEQ ID NO: 5: EGFR_L858R_F primer
SEQ ID NO: 6: EGFR_L858R_R primer
SEQ ID NO: 7: EGFR_L858R_sup_p3. 3'-end is modified by amino linker (NH2).
SEQ ID NO: 8: EGFR_L858R_P2. 5'-end is added Eclips and 3'-end is added FAM. nucleotide position 3 is RNA.
SEQ ID NO: 9: EGFR_19_delE746-A750_F primer
SEQ ID NO: 10: EGFR_19_delE746-A750_R primer
SEQ ID NO: 11: EGFR_19_delE746-A750 oligo-7. 3'-end is modified by amino linker (NH2)
SEQ ID NO: 12: EGFR_2236-2250del. 5'-end is added Eclips and 3'-end is added FAM. nucleotide position 3 is RNA.
SEQ ID NO: 13: k-rasG12_F primer
SEQ ID NO: 14: k-rasG12_R primer
SEQ ID NO: 15: k-rasG12/13_s1. 3'-end is modified by amino linker (NH2)
SEQ ID NO: 16: SEQ IDDNA fragment containing k-ras codon 12 and codon 13 regions
SEQ ID NO: 17: EGFRL858R_IC2_F primer
SEQ ID NO: 18: EGFRL858R_IC2_R primer
SEQ ID NO: 19: EGFRL858R_IC2_P2. 5'-end is added Eclips and 3'-end is added FAM. nucleotide position 3 is RNA.
SEQ ID NO: 20: EGFRL858R_IC2_P3. 5'-end is added Eclips and 3'-end is added FAM. nucleotide position 3 is RNA.

## Claims

1. A method of selectively cleaving a non-target nucleic acid in a sample containing a target nucleic acid and the non-target nucleic acid having a region of a nucleotide sequence homologous to the target nucleic acid, the method comprising a step of bringing the sample into contact with:
(i) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid; and
(ii) a polypeptide having a mismatch endonuclease activity.

2. The method according to claim 1, wherein the non-target nucleic acid has a nucleotide sequence which differs from the nucleotide sequence of the target nucleic acid by base substitution, and the oligonucleotide forms at least one mismatch when the oligonucleotide is hybridized with the non-target nucleic acid and forms a mismatch corresponding to the at least one mismatch and at least another mismatch when the oligonucleotide is hybridized with the target nucleic acid.

3. The method according to claim 2, wherein the oligonucleotide forms one mismatch when the oligonucleotide is hybridized with the non-target nucleic acid and forms a mismatch corresponding to the one mismatch and another mismatch when the oligonucleotide is hybridized with the target nucleic acid.

4. The method according to claim 3, wherein the two mismatches formed when the oligonucleotide is hybridized with the target nucleic acid are located contiguously or at an interval of not more than 5 bases.

5. The method according to claim 1, wherein the non-target nucleic acid has a nucleotide sequence which differs from the nucleotide sequence of the target nucleic acid by base insertion, and the oligonucleotide forms at least one mismatch when the oligonucleotide is hybridized with a region containing the insertion in the non-target nucleic acid.

6. The method according to claim 1, wherein the non-target nucleic acid has a nucleotide sequence which differs from the nucleotide sequence of the target nucleic acid by base deletion, and the oligonucleotide forms at least one mismatch when the oligonucleotide is hybridized with a region containing the deletion in the non-target nucleic acid.

7. The method according to any one of claims 1 to 6, wherein the polypeptide having a mismatch endonuclease activity is a polypeptide derived from a heat-resistant microorganism or a mutant thereof.

8. The method according to any one of claims 1 to 7, wherein the method is performed in the presence of an acidic high molecular substance.

9. A method of selectively amplifying a target nucleic acid in a sample containing the target nucleic acid and a non-target nucleic acid having a region of a nucleotide sequence homologous to the target nucleic acid, the method comprising:
(1) a step of cleaving the non-target nucleic acid in the sample by the method according to any one of claims 1 to 8; and
(2) a step of amplifying the target nucleic acid.

10. The method according to claim 9, wherein the amplification of the target nucleic acid is performed by PCR.

11. A method of selectively detecting a target nucleic acid in a sample containing the target nucleic acid and a non-target nucleic acid having a region of a nucleotide sequence homologous to the target nucleic acid, the method comprising:
(1) a step of selectively amplifying the target nucleic acid by the method according to claim 9 or 10; and
(2) a step of detecting the target nucleic acid simultaneously with or after step (1).

12. The method according to claim 11, wherein the detection of the target nucleic acid is performed by a cycling probe method or a TaqMan probe method.

13. A composition for the method according to any one of claims 9 to 12, containing:
(a) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid; and
(b) at least one pair of oligonucleotide primers;
(c) a polypeptide having a mismatch endonuclease activity; and
(d) a polypeptide having a DNA polymerase activity.

14. A kit for the method according to any one of claims 9 to 12, containing:
(a) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid;
(b) at least one pair of oligonucleotide primers;
(c) a polypeptide having a mismatch endonuclease activity; and
(d) a polypeptide having a DNA polymerase activity.

15. The composition according to claim 13 or the kit according to claim 14, further containing an acidic high molecular substance.

16. A method of selectively cleaving, in the presence of an acidic high molecular substance, a non-target nucleic acid in a sample containing a target nucleic acid and the non-target nucleic acid having a nucleotide sequence which differs from the nucleotide sequence of the target nucleic acid by at least one base, the method comprising a step of bringing the sample into contact with:
(i) the acidic high molecular substance;
(ii) a polypeptide having a mismatch endonuclease activity; and
(iii) an oligonucleotide which forms at least one mismatch when the oligonucleotide is hybridized with the non-target nucleic acid, and forms more mismatches when the oligonucleotide is hybridized with the target nucleic acid than when the oligonucleotide is hybridized with the non-target nucleic acid.


SEQUENCE LISTING

<110>  TAKARA BIO INC.

<120>   High sensitivity detection method of target nucleic acid

<130>  672822

<150>  JP2015-058270
<151>  2015-03-20

<160>  20

<170>  PatentIn version 3.5

<210>  1
<211>  17
<212>  DNA
<213>  Artificial Sequence

<220>
<223>  EGFR_T790M_F primer

<400>  1
atctgcctca cctccac                                                    17


<210>  2
<211>  19
<212>  DNA
<213>  Artificial Sequence

<220>
<223>  EGFR_T790M_R primer

<400>  2
caatattgtc tttgtgttc                                                  19


<210>  3
<211>  17
<212>  DNA
<213>  Artificial Sequence

<220>
<223>  T790M_NucG-1.  3'-end is modified by amino linker (NH2).

<400>  3
gctcatcagg cagctca                                                    17


<210>  4
<211>  10
<212>  DNA
<213>  Artificial Sequence

<220>
<223>  T790M_detect-1. 5'-end is added Eclips and 3'-end is added FAM.
       nucleotide position 4 is RNA.

<400>  4
catgcagctc                                                            10

<210>  5
<211>  20
<212>  DNA
<213>  Artificial Sequence

<220>
<223>  EGFR_L858R_F primer

<400>  5
aggaacgtac tggtgaaaac                                                    20


<210>  6
<211>  20
<212>  DNA
<213>  Artificial Sequence

<220>
<223>  EGFR_L858R_R primer

<400>  6
tccctggtgt caggaaaatg                                                    20


<210>  7
<211>  17
<212>  DNA
<213>  Artificial Sequence

<220>
<223>  EGFR_L858R_ sup_p3.  3'-end is modified by amino linker amino
       linker (NH2).

<400>  7
cagtttggtc agcccaa                                                       17


<210>  8
<211>  11
<212>  DNA
<213>  Artificial Sequence

<220>
<223>  EGFR_L858R_P2. 5'-end is added Eclips and 3'-end is added FAM.
       nucleotide position 3 is RNA.

<400>  8
ccgcccaaaa t                                                             11


<210>  9
<211>  21
<212>  DNA
<213>  Artificial Sequence

<220>
<223>  EGFR_19_delE746-A750_F primer

<400>  9
aacgtcttcc ttctctctct g                                                  21

<210>  10
<211>  20
<212>  DNA
<213>  Artificial Sequence

<220>
<223>  EGFR_19_delE746-A750_R primer

<400>  10
atcgaggatt tccttgttgg                                                    20


<210>  11
<211>  21
<212>  DNA
<213>  Artificial Sequence

<220>
<223>  EGFR_19_delE746-A750 oligo-7.  3'-end is modified by amino linker
       (NH2)

<400>  11
atgttgcttg tcttaattcc t                                                  21


<210>  12
<211>  11
<212>  DNA
<213>  Artificial Sequence

<220>
<223>  EGFR_2236-2250del. 5'-end is added Eclips and 3'-end is added
       FAM. nucleotide position 3 is RNA.

<400>  12
ctatcaagac a                                                             11


<210>  13
<211>  24
<212>  DNA
<213>  Artificial Sequence

<220>
<223>  k-rasG12_F primer

<400>  13
gtattaaaag gtactggtgg agta                                               24


<210>  14
<211>  23
<212>  DNA
<213>  Artificial Sequence

<220>
<223>  k-rasG12_R primer

<400>  14
gaattagctg tatcgtcaag gca                                                23


<210>  15

<211>  13
<212>  DNA
<213>  Artificial Sequence

<220>
<223>  k-rasG12/13_s1. 3'-end is modified by amino linker (NH2)

<400>  15
acgccagcag ctc                                                          13


<210>  16
<211>  73
<212>  DNA
<213>  Artificial Sequence

<220>
<223>  DNA fragment containing k-ras codon 12 and codon 13

<400>  16
atgactgaat ataaacttgt ggtagttgga gctggtggcg taggcaagag tgccttgacg      60

atacagctaa ttc                                                          73


<210>  17
<211>  24
<212>  DNA
<213>  Artificial Sequence

<220>
<223>  EGFRL858R_IC2_F primer

<400>  17
agcatcaaag gatggttcat gttt                                              24


<210>  18
<211>  24
<212>  DNA
<213>  Artificial Sequence

<220>
<223>  EGFRL858R_IC2_R primer

<400>  18
cctgtgtgtt tgccaatttt gtta                                              24


<210>  19
<211>  12
<212>  DNA
<213>  Artificial Sequence

<220>
<223>  EGFRL858R_IC2_P2. 5'-end is added Eclips and 3'-end is added FAM.
       nucleotide position 3 is RNA.

<400>  19
atgtctatgt tc                                                           12


<210>  20

<211>  11
<212>  DNA
<213>  Artificial Sequence

<220>
<223>  EGFRL858R_IC2_P3. 5′-end is added Eclips and 3′-end is added FAM.
       nucleotide position 3 is RNA.

<400>  20
tgaggggacg c                                                          11